Europäisches Patentamt

European Patent Office

Office européen des brevets

(19)

(11) **EP 0 637 451 B1**

(12) **EUROPÄISCHE PATENTSCHRIFT**

(45) Veröffentlichungstag und Bekanntmachung des Hinweises auf die Patenterteilung:
**13.10.1999 Patentblatt 1999/41**

(51) Int. Cl.$^6$: **A61K 38/36**, A61K 35/16

(21) Anmeldenummer: **94890132.7**

(22) Anmeldetag: **03.08.1994**

(54) **Virussichere Blutgerinnungsfaktor XIII-Präparation**

Preparation of blood coagulation factor XIII without viruses

Préparation de facteur XIII de coagulation du sang depourvue de tout virus

(84) Benannte Vertragsstaaten:
**AT BE CH DE DK ES FR GB IT LI NL SE**

(30) Priorität: **03.08.1993 AT 154893**

(43) Veröffentlichungstag der Anmeldung:
**08.02.1995 Patentblatt 1995/06**

(73) Patentinhaber:
**Baxter Aktiengesellschaft**
**1220 Wien (AT)**

(72) Erfinder: **Seelich, Thomas, Dr.**
**A-1030 Wien (AT)**

(74) Vertreter:
**Weinzinger, Arnulf, Dipl.-Ing. et al**
**Patentanwälte**
**Sonn, Pawloy, Weinzinger & Wolfram**
**Riemergasse 14**
**1010 Wien (AT)**

(56) Entgegenhaltungen:
**EP-A- 0 018 561**      **EP-A- 0 037 078**
**JP-A- 3 106 824**      **US-A- 5 610 147**

- **DATABASE WPI Week 9124, Derwent Publications Ltd., London, GB; AN 91-175118 & JP-A-3 106 824 (THE GREEN CROSS CORPORATION) 07 Mai 1991**
- **L.WINKELMAN ET AL.: "A pasteurised concentrate of human plasma factor XIII for therapeutic use", THROMBOSIS AND HAEMOSTASIS, , 1986, Band 55, Nr. 3, Seiten 402 - 405-**

**Beschreibung**

Die Erfindung betrifft eine virussichere Blutgerinnungsfaktor XIII-Präparation humaner Herkunft mit einem geringen Infektionsrisiko und gleichzeitig einer hohen Aktivität.

Das Proenzym Faktor XIII ist im Blutplasma in Form von a-und b-Ketten vorhanden ($a_2b_2$, Molekulargewicht ca. 320.000 Da). Faktor XIII der Formel $a_2$ (Molekulargewicht ca. 160.000 Da) findet sich beispielsweise in Plazenten und Thrombocyten. In beiden Varianten des Faktor XIII stellen die a-Ketten das eigentliche Proenzym dar, aus denen durch Einwirkung von Thrombin in Gegenwart von $Ca^{2+}$-Ionen das aktive Enzym, Faktor XIIIa, gebildet wird.

Faktor XIII spielt eine wesentliche Rolle in der letzten Phase der Blutgerinnung: Gleichzeitig mit der durch Thrombin bewirkten Umwandlung von Fibrinogen in Fibrin (Gerinnung) wird Faktor XIII in Gegenwart von $Ca^{2+}$-Ionen zu Faktor XIIIa aktiviert, der seinerseits die gebildeten Fibrin-Monomere kovalent vernetzt. Das solcherart entstandene Hochpolymere weist eine wesentlich höhere mechanische Festigkeit auf als nicht vernetztes Fibrin und ist auch resistenter gegenüber fibrinolytischem Abbau, was für eine wirksame Blutstillung von entscheidender Bedeutung ist. Darüber hinaus ist die Anwesenheit von Faktor XIII eine notwendige Voraussetzung für den normalen Verlauf der Wundheilung.

Faktor XIII-Mangelpatienten leiden nach Verletzungen an schweren Blutstillungs- und Wundheilungsstörungen, die nur durch intravenöse Gabe von Faktor XIII-Präparationen behoben werden können. Aber auch in Gewebeklebstoffen auf Basis von Fibrinogen und Faktor XIII ("Fibrinkleber") ist ein ausreichender Gehalt an Faktor XIII wesentlich, um die erwünschte hohe Festigkeit der Klebungen, wirksame Blutstillung und gute Wundheilung zu erreichen.

Schließlich besteht für medizinisch-diagnostische Zwecke ein Bedarf an standardisierten Faktor XIII-Präparationen.

Faktor XIII-Präparationen, insbesondere zur Anwendung am Menschen, werden aus Humanplasma und aus humanen Plazenten gewonnen. Aufgrund des verwendeten Ausgangsmaterials besteht grundsätzlich das Risiko der Übertragung infektiöser Agentien, wie Viren, die durch Sterilfiltration nicht entfernt werden können.

Für alle erwähnten Anwendungen besteht die Forderung, daß die Faktor XIII-Präparationen praktisch kein Infektionsrisiko aufweisen dürfen, um weder Patienten noch Personal zu gefährden. Im besonderen Maß besteht diese Forderung bei Präparationen zur Anwendung am Menschen, und in höchstem Maß bei Infusionspräparaten, die beispielsweise Faktor XIII-Mangelpatienten das ganze Leben hindurch in regelmäßigen Abständen verabreicht werden müssen. Denn diese wiederholte Gabe würde bei allen Präparaten, die nicht absolut frei von infektiösen Partikeln sind, zu einer Akkumulation dieser Partikel im Patienten und somit zu einem wesentlich erhöhten Infektionsrisiko führen.

Man hat daher versucht, dieses Risiko nach Möglichkeit zu minimieren, und zwar einerseits durch Testung des Ausgangsmaterials und andererseits durch zusätzliche Maßnahmen zur Inaktivierung eventuell vorhandener infektiöser Agentien. Eine Testung des Ausgangsmaterials ist jedoch nur auf bereits bekannte infektiöse Agentien, für die auch geeignete Testmethoden zur Verfügung stehen, möglich (Hepatitis-, HI-Viren); nicht jedoch auf andere, möglicherweise vorhandene unbekannte infektiöse Agentien. Darüber hinaus ist die Empfindlichkeit aller Testmethoden begrenzt, so daß schon allein deshalb keine absolute Sicherheit zu erwarten ist.

Allen Verfahren zur Inaktivierung von Krankheitserregern, wie Viren in empfindlichen biologischen Präparaten, liegt das Problem zugrunde, daß durch das Verfahren einerseits eine möglichst große Reduktion des Virustiters, andererseits eine möglichst geringe Abnahme der biologischen Aktivität erfolgen soll.

Virusinaktivierungsverfahren werden heutzutage als wirksam bezeichnet, wenn nach Anwendung des Verfahrens an einer Test-Präparation, welche mit einer hohen Dosis eines Testvirus versetzt wurde (z.B. entsprechend einem maximal möglichen Titer von ca. $10^5$ ID/ml in einer Gerinnungsfaktor-Präparation) keine Viren mehr in der Probe nachgewiesen werden können und der Virustiter somit unter die Nachweisgrenze reduziert wurde.

Als Maß für die Inaktivierung ist der sogenannte "Reduktionsfaktor" ("virus reduction factor") bekannt, der nach einer einmaligen Zugabe von Testvirus aus dem dekadischen Logarithmus des Quotienten von Anfangs- und Endvirustiter errechnet wird. Aus der Richtlinie EG III/8115/89-EN der Kommission der Europäischen Gemeinschaften ist weiters der sogenannte Gesamt-Reduktionsfaktor bekannt. Er wird aus der Summe der Reduktionsfaktoren von einzelnen subsequenten Inaktivierungsmaßnahmen berechnet.

Als Maß für den praktischen Wert von Virusinaktivierungsverfahren dient daher das Verhältnis von Reduktionsfaktor (RI) bzw. Gesamtreduktionsfaktor (R) zur biologischen Restaktivität (RA) nach Durchführung des Verfahrens.

Verfahren zur Inaktivierung von Krankheitserregern in Faktor XIII-Präparaten durch Erhitzen in Lösung (Pasteurisierung) sind bereits bekannt (EP-A-0 018 561, EP-A-0 037 078). Die biologische Aktivität des Faktor XIII kann durch den Zusatz von bekannten Stabilisatoren, wie Aminosäuren, Peptiden, Zuckern, Polyolen oder Carbonsäuren, sogenannten "Thermostabilisatoren", die in einer Menge von mehr als 10% bis 50% (Sirupverfahren) eingesetzt werden müssen, im wesentlichen erhalten werden. Weiters wurde auch Humanalbumin in einer Konzentration von mehr als 0,1% als Thermostabilisator vorgeschlagen (JP 80960/1988). Ebenso ist ein Verfahren zur Stabilisierung von Blutgerinnungsfaktoren durch Zusatz von mehr als 0,5 M Ammoniumsulfat vor einer Wärmebehandlung bekannt (AT 376 883).

Die notwendige Entfernung der hoch konzentrierten Thermostabilisatoren aus den erhitzten Produkten ist jedoch aufwendig. Virusinaktivierungsverfahren durch Pasteurisierung in Gegenwart von Stabilisatoren haben weiters den Nachteil, daß gleichzeitig mit der erwünschten biologischen Aktivität auch die Viren selbst stabilisiert werden, wodurch kein besonders günstiges Verhältnis von Reduktionsfaktor zu Restaktivität (R/RA) erhalten wird.

Eine Flüssig-Erhitzung von Faktor XIII-hältigen Lösungen ohne Stabilisatoren erschien nach fächmännischer Meinung bisher unmöglich; so wurde in einem Vergleichsbeispiel in der EP-A-0 037 078 Faktor XIII ohne Zusatz von Stabilisatoren 10 h bei 60°C erhitzt. Die Aktivität von Faktor XIII wurde dabei vollständig zerstört.

Die Erfindung stellt sich die Aufgabe, eine virussichere Blutgerinnungsfaktor XIII-Präparation zur Verfügung zu stellen, von der sowohl erwartet werden kann, daß die Übertragung von infektiösen Agentien selbst bei Verabreichung von großen Mengen an Faktor XIII ausgeschlossen ist, und welche trotzdem noch eine hohe Aktivität besitzt. Die Erfindung stellt sich weiters die Aufgabe, ein Verfahren zur Inaktivierung von infektiösen Agentien in einer Faktor XIII-hältigen Präparation durch Erhitzen in Lösung zur Verfügung zu stellen, welches die Nachteile der bekannten Pasteurisierungsverfahren nicht aufweist.

Diese Aufgaben werden erfindungsgemäß gelöst durch eine virussichere Blutgerinnungsfaktor XIII-Präparation, erhältlich durch Erhitzen einer wässerigen Lösung enthaltend den Blutgerinnungsfaktor XIII mit einer spezifischen Aktivität von mindestens 2 E/mg Gesamtprotein, welche Lösung weniger als 10% an bekannten Stabilisatoren, ausgesucht aus der Gruppe der Zucker, Polyole, Aminosäuren, Peptide und Carbonsäuren, sowie weniger als 0,5 Mol Ammoniumsulfat pro Liter enthält, wobei das Erhitzen während einer Zeitdauer vorgenommen wird, die ausreicht, infektiöse Agentien zu inaktivieren, vorzugsweise während 30 min bis 100 h.

Die Erfindung umfaßt weiters ein Verfahren zur Herstellung einer virussicheren Blutgerinnungsfaktor XIII-Präparation, wobei infektiöse Agentien durch Erhitzen inaktiviert werden, welches dadurch gekennzeichnet ist, daß eine wässerige Lösung einer Faktor XIII-hältigen Fraktion, die weniger als 10% an bekannten Stabilisatoren, ausgesucht aus der Gruppe der Zucker, Polyole, Aminosäuren, Peptide und Carbonsäuren, sowie weniger als 0,5 Mol Ammoniumsulfat pro Liter enthält und eine spezifische Aktivität von mindestens 2, vorzugsweise mindestens 15, Einheiten/mg Gesamtprotein aufweist, bei einer Temperatur von 40 bis 65°C während einer Zeitdauer, die ausreicht, infektiöse Agentien zu inaktivieren, vorzugsweise während 30 min bis 100 h, erhitzt wird.

Es hat sich überraschenderweise gezeigt, daß beim Erhitzen von Faktor XIII-hältigen Lösungen in Abwesenheit von bzw. bei einem geringen Gehalt an bekannten Stabilisatoren ein äußerst günstiges, bisher nicht erreichtes Verhältnis von Virus-Reduktionsfaktor zu Faktor XIII-Restaktivität erhalten wird. Vergleichsversuche zeigen, daß eine bestimmte Reduktion des HIV-Titers durch Erhitzen in Lösung (60°C) eine mindestens zehnmal längere Erhitzungsdauer erfordert, wenn die Erhitzung in Gegenwart von Stabilisatoren (50% Saccharose, 2 M Glycin) entsprechend der EP-A-0 018 561) durchgeführt wird.

Die weitgehende Erhaltung der Faktor XIII-Aktivität war umso überraschender, da der Faktor XIII als relativ hitzelabil gilt, wenn dieser beispielsweise einer Hitzebehandlung gemäß der EP-B-0 159 311 unterzogen wird.

Das erfindungsgemäße Verfahren wird bei einer Temperatur und einer Dauer durchgeführt, bei der die biologische Aktivität des Faktor XIII zu mehr als 50% erhalten bleibt.

Eine bevorzugte Ausführungsform des Verfahrens ist gekennzeichnet durch die folgenden Maßnahmen:

-    Bereitstellen einer Faktor XIII-hältigen COHN I-Fraktion,
-    Fällen von Faktor XIII aus der COHN I-Fraktion und Abtrennen des Niederschlages,
-    gegebenenfalls weiteres Reinigen des Faktor XIII-hältigen Niederschlages durch Lösen und Umfällen,
-    kurzzeitiges Erhitzen des gelösten Niederschlages und Abtrennen des hitzedenaturierten Fibrinogens,
-    gegebenenfalls weiteres Reinigen und Ankonzentrieren der Faktor XIII-hältigen Lösung,
-    Erhitzen der Faktor XIII-hältigen Lösung ohne Zusatz von bekannten Stabilisatoren bei einer Temperatur von 40 bis 60°C während einer Dauer von 30 min bis 2 h.

Die Fällung des Faktor XIII aus der gelösten COHN I-Fraktion kann beispielsweise durch Ammoniumsulfat (etwa 10- bis 20%ige Sättigung bei 20°C) oder durch Glycin (etwa 1,5 bis 3 Mol/l) erfolgen. Auch andere Fällungsmittel, die bekannterweise zur Ausfällung von Faktor XIII (in Gegenwart von Fibrinogen) geeignet sind, können verwendet werden.

Nach Abtrennen und Lösen des erhaltenen Niederschlages kann die Fällung mit demselben oder einem anderen Fällungsmittel wiederholt werden. Der Niederschlag wird dann gelöst und kurz erhitzt (beispielsweise 3 min bei 56°C), um vorhandenes Fibrinogen zu denaturieren und auszufällen. Der Hitzefällungsüberstand hat einen Gesamtproteingehalt von etwa 0,5 bis 2,5 mg/ml und enthält Faktor XIII mit einer spezifischen Aktivität von etwa 2 bis 10 E/mg Gesamtprotein.

Diese Faktor XIII-hältige Lösung kann dadurch weiter gereinigt werden, daß Begleitproteine durch Zugabe von Polyethylenglykol (z.B. PEG 4000 bis zu einer Konzentration von etwa 3,5% bei etwa 4°C) ausgefällt und abgetrennt werden.

Danach kann der Faktor XIII durch Zugabe von PEG 4000 bis zu einer Konzentration von 10% ausgefällt und in einer Pufferlösung, beispielsweise einer 0,1%igen Citratlösung, pH 7,0, zu einer Konzentration von mehreren hundert Einheiten pro ml gelöst werden.

Die so erhaltene Lösung wird dann ohne Zusatz von bekannten Stabilisatoren erhitzt (z.B. 2 Stunden bei 60°C bzw. 12 Stunden bei 50°C), wobei die Faktor XIII-Aktivität im wesentlichen erhalten bleibt.

In einer bevorzugten Ausführungsform enthält die zu erhitzende Lösung zusätzlich ein Tensid, vorzugsweise ein nichtionisches Tensid. Die Tensidkonzentration ist je nach Art des Tensides unterschiedlich zu wählen. Zweckmäßig ist eine Konzentration, in der das Tensid während der Erhitzung zusätzlich viruzid wirksam ist, aber die Faktor XIII-Aktivität nicht wesentlich beeinträchtigt wird.

Eine solche zweckmäßige Konzentration kann für jedes gewünschte Tensid auf einfache Weise experimentell ermittelt werden.

Beispielsweise liegen zweckmäßige Konzentrationen für Desoxycholat unter 0,2% (w/v), Benzyltrimethylammoniumchlorid und Benzyldimethyl-2-hydroxyäthylammoniumchlorid unter 0,1% (w/v), Sulfobetain SB 12 (Fa. Serva; N-Dodecyl-N', N-dimethyl-ammonio-1-propansulfonat) unter 0,3% (w/v), und N-Octylglucosid unter 1% (w/v), während Tenside wie Tween 80 oder Triton X-100 auch in wesentlich höheren Konzentrationen [15% (w/v) und darüber] eingesetzt werden können.

Es hat sich überraschenderweise gezeigt, daß die Stabilität des Faktor XIII beim Erhitzen in Lösung in Gegenwart von Tensiden etwa ebenso gut ist wie in deren Abwesenheit, die Virusinaktivierung jedoch noch schneller verläuft, so daß insgesamt ein noch günstigeres Verhältnis von Virus-Reduktionsfaktor zu Faktor XIII-Restaktivität erhalten wird. Erforderlichenfalls kann das zugesetzte Tensid nach dem Erhitzungsschritt durch an sich bekannte Methoden, wie z.B. Umfällen oder Ionenaustausch-Chromatographie, wieder entfernt werden.

Die erfindungsgemäße Präparation eignet sich durch ihre bisher nicht erreichte Sicherheit gegenüber der Übertragung infektiöser Agentien, wie Hepatitis- oder AIDS-Viren (HIV), bei gleichzeitig hoher spezifischer Enzymaktivität für die Herstellung von Präparaten zur prophylaktischen und therapeutischen Anwendung am Menschen, für die Herstellung von Gewebeklebstoffen sowie für diagnostische Zwecke.

Eine bevorzugte Verwendung der virussicheren Blutgerinnungsfaktor XIII-Präparation besteht in der Herstellung eines virussicheren Gewebeklebstoff-Präparates als Zusatz zu einem bereits virusinaktivierten Fibrinogen-Präparat.

Die Dauer der erfindungsgemäßen Wärmebehandlung zur Erreichung eines gewünschten Reduktionsfaktors kann experimentell an einer Faktor XIII-Probe, der eine bestimmte Menge Testvirus zugesetzt wurde, ermittelt werden.

Die Faktor XIII-Probe wird zur Virusinaktivierung so lange bei einer bestimmten Temperatur behandelt, bis ein gerade noch meßbarer Virustiter erreicht wird. Aus der Reduktion des Virustiters pro Zeiteinheit kann die Dauer der Behandlung errechnet werden, die notwendig ist, um eine gewünschte Gesamtreduktion des Virustiters zu erreichen.

Bei dieser Berechnungsweise wird vorausgesetzt, daß die Virusinaktivierung eine Reaktion 1. Ordnung mit gleichbleibender Geschwindigkeitskonstante ist.

Auch wenn dies in der Praxis nicht immer der Fall ist, sondern öfters eine Abnahme der Wirksamkeit eines Verfahrens mit dessen Dauer beobachtet wird (sogen. tailing), so ist die beschriebene Berechnungsweise dennoch eine anerkannte Methode, um die Wirksamkeit verschiedener Virusinaktivierungsverfahren miteinander zu vergleichen.

Es ist aber auch möglich, während der Hitzebehandlung bestimmte Mengen Testvirus nach dem Verfahren der EP 0 519 901 wiederholt zuzusetzen, wobei jede Wiederholung erst dann vorgenommen wird, wenn der Virustiter auf einen bestimmten Wert, vorzugsweise unter die Nachweisgrenze, abgesunken ist. Der Gesamtreduktionsfaktor ergibt sich dann aus der Summe der einzelnen Reduktionsfaktoren.

Es hat sich gezeigt, daß zur Durchführung des erfindungsgemäßen Verfahrens eine Fraktion enthaltend hochgereinigten Faktor XIII besonders geeignet ist. Dementsprechend besteht eine bevorzugte Ausführungsform des erfindungsgemäßen Verfahrens darin, daß vor der Hitzeinaktivierung die Faktor XIII-hältige Fraktion gereinigt und gegebenenfalls konzentriert wird, um eine spezifische Aktivität des Faktor XIII von mindestens 15 Einheiten pro mg Gesamtprotein zu erreichen.

Es war überraschend, daß die Stabilität des Faktor XIII bei Durchführung des erfindungsgemäßen Verfahrens umso besser ist, je weiter die Präparation gereinigt wurde, d.h. je höher die spezifische Aktivität ist.

Es wäre nämlich das Umgekehrte zu erwarten gewesen, da im allgemeinen empfindliche Enzyme durch die Anwesenheit anderer Proteine (ein bekanntes Beispiel ist Albumin) stabilisiert werden.

Überraschenderweise wurde jedoch gefunden, daß bei dem erfindungsgemäßen Verfahren Albumin keine stabilisierende Wirkung auf Faktor XIII hat, und auch andere Plasmaproteine nicht stabilisierend wirken.

Es ist vorteilhaft, die Faktor XIII-hältige Fraktion aus Plasma zu gewinnen. Faktor XIII besteht dann sowohl aus a- als auch aus b-Ketten.

Es ist vorteilhaft, eventuell vorhandenes Fibrinogen vor Durchführung des erfindungsgemäßen Verfahrens abzutrennen. In Anwesenheit von hitzedenaturiertem Fibrinogen könnte es zu Einschlüssen von Viren kommen. Solcherart eingeschlossene Viren könnten gegenüber Hitzeeinwirkung stabilisiert werden und dadurch die Verläßlichkeit des Verfahrens in Frage stellen. Fibrinogen wird nach kurzzeitigem Erhitzen (etwa einige Minuten bei 56°C bis 60°C) denaturiert und kann dann als Präzipitat abgetrennt werden.

Das erfindungsgemäße Verfahren kann in jeder Stufe des Plasmafraktionierungsverfahrens durchgeführt werden, in der der Faktor XIII mit einer spezifischen Aktivität von wenigstens 2 Einheiten pro mg Gesamtprotein in Lösung vorliegt.

In den nachfolgenden Beispielen wurden die folgenden analytischen Methoden angewendet:

Gesamtprotein: Biuret-Methode

Faktor XIII: Die Bestimmung erfolgte nach zwei verschiedenen Methoden, die jedoch beide auf demselben Grundprinzip, nämlich der Vernetzung von Fibrin durch Faktor XIII, beruhen.

Methode 1:

Aliquots einer Faktor XIII-freien Fibrinogenlösung werden mit verschiedenen Verdünnungen der zu bestimmenden Probe und einer Thrombin-CaCl$_2$-Lösung vermischt und bei 37°C inkubiert. Nach einer bestimmten Inkubationszeit wird die Reaktion durch Zugabe von 1%iger Monochloressigsäure (MCE) gestoppt und die Löslichkeit bzw. Unlöslichkeit der Fibrinclots in MCE festgestellt.

Als Standard dient gepooltes tiefgefrorenes humanes Citratplasma, wobei per definitionem 1 ml Plasma 1 Einheit Faktor XIII enthält.

Die jeweils höchsten Verdünnungen von Probe und Standard, mit denen eben noch unlösliche Clots erhalten werden, dienen zur Berechnung des Faktor XIII-Gehaltes der unbekannten Probe (x) nach der Formel

$$x = \frac{V_x}{V_s} \text{ , wobei}$$

$V_x$     die Verdünnung der unbekannten Probe und
$V_s$     die Verdünnung des Standards ist.

Diese allgemein übliche Bestimmungsmethode beruht also letztlich auf der Löslichkeit von unvernetztem bzw. der Unlöslichkeit von vernetztem Fibrin in 1%iger Monochloressigsäure. Ihre Genauigkeit ist daher durch die Unschärfe dieser Löslichkeitsgrenze limitiert.

Methode 2:

Die Reaktionsansätze entsprechen denjenigen von Methode 1, jedoch wird die Reaktion durch Zugabe eines Gemisches von Harnstoff, Na-Dodecylsulfat (SDS) und β-Mercaptoäthanol gestoppt und werden die in den Proteinen enthaltenen Disulfidbrücken reduktiv gespalten.

Der Vernetzungsgrad der Fibrin-γ-Ketten in den so erhaltenen Proben wird nach SDS-Gelelektrophorese und Färbung mit Coomassie-Blau densitometrisch bestimmt. Durch graphische Interpolation erhält man daraus diejenigen Verdünnungen von Probe und Standard, bei denen die Vernetzung der Fibrin-γ-Ketten 50% beträgt. Betragen diese Verdünnungen $V_x$ bzw. $V_s$, so erhält man den Faktor XIII-Gehalt der unbekannten Probe (x) wie in Methode 1 nach der Formel

$$x = \frac{V_x}{V_s} \text{ .}$$

Methode 2 ist naturgemäß genauer als die (allgemein übliche) Methode 1, jedoch auch wesentlich arbeitsaufwendiger.

Methode 2 wurde daher dann angewendet, wenn eine höhere Genauigkeit der Faktor XIII-Bestimmung zur Beschreibung des erfindungsgemäßen Verfahrens vorteilhaft oder notwendig schien.

Beispiel 1:

Herstellung einer fibrinogenfreien Faktor XIII-Lösung (Hitzefällungsüberstand)

Ein herkömmlicher COHN I-Niederschlag (erhalten aus einer Fibrinogen und Faktor XIII-enthaltenden Plasmafraktion durch Fällung mit Ethanol) wurde mit der 10-fachen Menge einer citrathältigen Pufferlösung pH 7,0 (0,05 M Citrat, 0,5 M NaCl, 20.000 KIE Aprotinin/l) gelöst und unter Rühren mit Ammoniumsulfat bis zur 16%igen Sättigung (bei Raumtemperatur) versetzt. Danach wurde auf 4°C abgekühlt und das Gemisch noch 2 Stunden gerührt. Der entstandene Niederschlag wurde mit der beschriebenen Pufferlösung gelöst und die Fällung mit Ammoniumsulfat einmal wiederholt.

Der Niederschlag wurde in einer citrathältigen Pufferlösung pH 7,0 (0,02 M Citrat, 0,12 M NaCl, 100 KIE Aprotinin/ml) gelöst und 10 min auf 56°C erhitzt. Der entstandene Niederschlag (aus denaturiertem Fibrinogen) wurde abzentrifugiert. Der Hitzefällungsüberstand hatte einen Proteingehalt von 2,1 mg/ml und einen Faktor XIII-Gehalt (nach Methode 2) von 7,7 E/ml. Die spezifische Aktivität betrug daher 3,7 E Faktor XIII/mg Protein.

Beispiel 2:

Stabilität des Faktor XIII beim Erhitzen der Lösung

Hitzefällungsüberstände entsprechend Beispiel 1 wurden bei Temperaturen von 40 bis 65°C ohne Zusatz von Stabilisatoren erhitzt. Nach einer bestimmten Dauer der Wärmebehandlung wurde die Restaktivität des Faktor XIII, bezogen auf die Aktivität vor dem Erhitzen, bestimmt.

Tabelle 1

| Erhitzen von Hitzefällungsüberstand ohne Zusatz von Stabilisatoren | | | |
|---|---|---|---|
| Temp. | Dauer (h) | Restaktivität (%) | bestimmt nach Methode |
| 40°C | 7 | 100 | 1 |
| | 24 | 80 | 1 |
| 50°C | 7 | 100 | 1 |
| | 24 | 80 | 1 |
| 60°C | 1 | 85 | 2 |
| | 2 | 73 | 2 |
| | 4 | 50 | 2 |
| 65°C | 0,5 min | 85 | 1 |
| | 1 min | 75 | 1 |
| | 2 min | 55 | 1 |

Beispiel 3:

Spezifische Aktivität und Stabilität des Faktor XIII beim Erhitzen in Lösung

Die Stabilität beim Erhitzen in Lösung ohne Stabilisatoren folgender Faktor XIII-Lösungen wurde verglichen:

A) Der Hitzefällungsüberstand aus Beispiel 1

B) Der Ammoniumsulfatniederschlag aus Beispiel 1 wurde zur weiteren Reinigung in einer citrathältigen Pufferlösung (0,02 M Citrat, 0,12 M NaCl, 50.000 KIE Aprotinin/l) gelöst und mit Glycin (1,75 Mol/l) umgefällt. Der Niederschlag wurde in gleicher Pufferlösung gelöst und die Hitzefällung entsprechend Beispiel 1 durchgeführt.

C) Ein Hitzefällungsüberstand entsprechend Beispiel 1 wurde zur weiteren Reinigung und Konzentrierung folgendermaßen weiterbehandelt:

1) Abtrennung von Begleitproteinen durch Fällung mit 3,5% (w/v) PEG 4000 bei 4°C

2) Totalfällung des Faktor XIII aus dem Überstand durch Zugabe von PEG 4000 bis zu einer Konzentration von 10% (w/v) bei 4°C, Abtrennen des Niederschlages durch Zentrifugation und Lösen in 1/25 des ursprünglichen Volumens eines 0,1%igen Na-Citratpuffers, pH 7,0

Die Faktor XIII-Restaktivität der drei Lösungen A, B, C nach der Wärmebehandlung (4 Stunden 60°C ohne Stabilisatoren) wurde bestimmt (Methode 2).

Tabelle 2

| Erhitzen von Faktor XIII-haltigen Lösungen unterschiedlicher Reinheit | | |
|---|---|---|
| Lösung | spez. Aktivität (E/mg Gesamtprotein) | Restaktivität (%) nach 4 h 60°C |
| A) | 3,7 | 50 |
| B) | 17 | 82 |
| C) | 35 | 95 |

Das Beispiel zeigt, daß die Stabilität des Faktor XIII beim Erhitzen in Lösung ohne Stabilisatoren mit dessen Reinheit (spezifischer Aktivität) zunimmt.

Beispiel 4:

Stabilität des Faktor XIII beim Erhitzen in Lösung (ohne Stabilisatoren) in Gegenwart eines Tensides

Eine Faktor XIII-hältige Lösung wurde entsprechend Beispiel 3, Variante C, hergestellt. Die spezifische Aktivität betrug 21 E Faktor XIII/mg Protein. Die Lösung wurde geteilt und ein Teil mit 1% (w/v) Tween 80 versetzt. Beide Lösungen wurden 6 Stunden lang auf 60°C erhitzt. Die Faktor XIII-Restaktivitäten nach 6-stündiger Erhitzung betrugen 82% ohne Tensidzusatz und 84% mit Tensidzusatz (Bestimmung nach Methode 2).

Beispiel 5:

Beispiel 4 wurde mit verschiedenen Tensiden in unterschiedlichen Konzentrationen wiederholt (Erhitzung: 4 Std. 60°C).

Tabelle 3

| Erhitzen einer Faktor XIII-haltigen Lösung in Anwesenheit von Tensid | | |
|---|---|---|
| Tensid | Konzentration % (w/v) | FXIII-Restaktivität % |
| Tween 80 | 15 | 97 |
| Triton X-100 | 15 | 91 |
| Pluronic P 85 | 10 | 96 |
| N-Octylglucosid | 0,3 | 86 |
| Na-Desoxycholat | 0,1 | 67 |
| Benzyltrimethylammoniumchlorid | 0,01 | 97 |
| Benzyldimethyl-2-hydroxyäthylammoniumchlorid | 0,01 | 93 |
| Sulfobetain SB 12 | 0,03 | 93 |
|  | 0,1 | 63 |

Die Beispiele 4 und 5 zeigen, daß das erfindungsgemäße Verfahren ohne weiteres auch in Gegenwart von nichtionischen, anionischen, kationischen oder zwitterionischen Tensiden durchgeführt werden kann, ohne größere Verluste an Faktor XIII-Aktivität in Kauf nehmen zu müssen.

Die folgenden Beispiele 6 und 7 zeigen die Wirksamkeit des erfindungsgemäßen Verfahrens.

Beispiel 6:

Inaktivierung von Modellviren nach dem erfindungsgemäßen Verfahren in Anwesenheit bzw. Abwesenheit von denaturiertem Fibrinogen

Proben Faktor XIII-hältiger Fraktionen entsprechend Beispiel 1, entweder

A) vor Hitzefällung und Abtrennung des Fibrinogens oder
B) nach Abtrennung des Hitzefällungsniederschlages, wurden mit 10 Vol % von Suspensionen von Sindbis-, Vaccinia-, Polio-, oder Vesicular Stomatitis (VS)-Virus versetzt und auf 60°C erhitzt, der Virustiter nach unterschiedlichen Erhitzungszeiten bestimmt und der Reduktionsfaktor (R) bezogen auf 60 min Erhitzungsdauer berechnet. In Variante A) erfolgte die Virustiterbestimmung analog zu B) aus den während der Erhitzung gebildeten Hitzefällungsüberständen.

Die Ergebnisse sind in der Tabelle 4 zusammengestellt.

Tabelle 4

| Abnahme des Virustiters in einer Faktor XIII-hältigen Lösung in Anwesenheit oder in Abwesenheit von denaturiertem Fibrinogen (Variante A bzw. B) Virustiter (log10) nach Minuten bei 60°C | | | | | | | | | | |
|---|---|---|---|---|---|---|---|---|---|---|
| Virus | Variante | 0 | 5 | 10 | 15 | 20 | 30 | 45 | 60 | R |
| Sindbis | A | 6,5 | - | ≤0,5 | - | ≤0,5 | ≤0,5 | ≤0,5 | ≤0,5 | ≥36 |
| Vaccinia | | 6,2 | - | 2,5 | - | 0,7 | ≤0,5 | ≤0,5 | ≤0,5 | 16,5 |
| Polio | | 7,0 | - | ≤0,5 | - | ≤0,5 | ≤0,5 | ≤0,5 | ≤0,5 | ≥39 |
| Sindbis | B | 6,5 | - | ≤0,5 | - | ≤0,5 | ≤0,5 | ≤0,5 | ≤0,5 | ≥36 |
| Sindbis | | 6,4 | 1,2 | ≤0,5 | ≤0,5 | - | ≤0,5 | - | ≤0,5 | 62 |
| Vaccinia | | 6,2 | - | 1,0 | - | ≤0,5 | ≤0,5 | ≤0,5 | ≤0,5 | 31,2 |
| Polio | | 7,0 | - | ≤0,5 | - | ≤0,5 | ≤0,5 | ≤0,5 | ≤0,5 | ≥39 |
| VSV | | 7,0 | 0,7 | ≤0,5 | ≤0,5 | - | ≤0,5 | - | ≤0,5 | 54 |

Die Ergebnisse zeigen die ausgezeichnete Wirksamkeit des Verfahrens im Vergleich zu dem nächstliegenden bekannten Verfahren, nämlich Pasteurisierung in Gegenwart von Stabilisatoren.
Die Wirksamkeit dieses bekannten Verfahrens ist z.B. aus R. Mauler u. J. Hilfenaus, Arzneim.Forsch./Drug res. 34, 1524-1527, 1984, Tab. 2, S 1526, bekannt.
Berechnet man die entsprechenden Reduktionsfaktoren (1 Stunde 60°C) für Polio- u. Vaccinia-Virus, und bestimmt (durch graphische Interpolation) diejenigen Erhitzungszeiten (60°C), nach denen eine Virustiter-Reduktion um $10^5$ eintritt (R=5), so ergibt sich folgender Vergleich (Tab. 5):
Die Ergebnisse (Tab. 4) zeigen weiters am Beispiel von Vaccinia-Virus, daß es vorteilhaft ist, ausgefallenes denaturiertes Protein vor dem Flüssigerhitzungsschritt zu entfernen.

Tabelle 5

| Vergleich der Virusinaktivierungskapazität des erfindungsgemäßen Verfahrens mit einem Verfahren des Standes der Technik | | | |
|---|---|---|---|
| Verfahren | Virus | R (1h 60°C) | Erhitzungsdauer 60°C (R = 5) |
| Stand d.Technik (Mauler et al) | Polio | 1,2 | 4h 10 min |
| | Vaccinia | 1,75 | 2h 52 min |

# EP 0 637 451 B1

Tabelle 5 (fortgesetzt)

| Vergleich der Virusinaktivierungskapazität des erfindungsgemäßen Verfahrens mit einem Verfahren des Standes der Technik | | | |
|---|---|---|---|
| Verfahren | Virus | R (1h 60°C) | Erhitzungsdauer 60°C (R = 5) |
| Erfindungsgemäßes Verf. | Polio | $\geq 39$ | $\leq 8$ min |
| | Vaccinia | 31 | 10 min |

Beispiel 7:

Inaktivierung eines Modellvirus (Sindbis) nach dem erfindungsgemäßen Verfahren in Anwesenheit bzw. Abwesenheit eines Tensides.

Eine Faktor XIII-haltige Lösung entsprechend Beispiel 3, Variante C wurde geteilt und ein Teil mit 0,3% (w/v) N-Octylglukosid versetzt.

Beide Lösungen wurden auf 60°C erhitzt, mit 10 Vol % einer Sindbis-Virus-Suspension versetzt (Start der Virusinaktivierungsreaktion) und bei 60°C weiter inkubiert. In bestimmten Zeitabständen wurden Proben gezogen und der Virustiter bestimmt.

Die Ergebnisse sind in der folgenden Tabelle 6 zusammengestellt.

Tabelle 6

| Virusinaktivierung in einer Faktor XIII-haltigen Lösung mit und ohne Tensid bei 60°C | | |
|---|---|---|
| | Virustiter (log10) | |
| Dauer des Erhitzens auf 60°C (Minuten) | ohne Tensid | mit Tensid |
| 0 | 8,1 | 8,1 |
| 0,5 | 5,75 | 1,88 |
| 1 | 5,0 | $\leq 1,5$ |
| 1,5 | 4,25 | $\leq 1,5$ |
| 2 | 3,88 | $\leq 1,5$ |
| 2,5 | 3,75 | $\leq 1,5$ |
| 3 | 3,25 | $\leq 1,5$ |
| 3,5 | 2,75 | $\leq 1,5$ |
| 4 | 2,63 | $\leq 1,5$ |
| 4,5 | 2,5 | $\leq 1,5$ |
| 5 | 2,38 | $\leq 1,5$ |
| 6 | 1,88 | $\leq 1,5$ |
| 7 | 2,1 | $\leq 1,5$ |
| 8,10,15,20,30 | $\leq 1,5$ | $\leq 1,5$ |

Das Beispiel zeigt, daß das erfindungsgemäße Verfahren noch wirksamer ist, wenn es in Gegenwart eines Tensides durchgeführt wird:

Es wird eine noch schnellere und weitgehendere Virusinaktivierung erhalten, ohne größere Verluste an Faktor XIII-Aktivität in Kauf nehmen zu müssen (vgl. Beispiel 5).

## Patentansprüche

1.  Virussichere Blutgerinnungsfaktor XIII-Präparation, erhältlich durch Erhitzen einer wässerigen Lösung enthaltend den Blutgerinnungsfaktor XIII mit einer spezifischen Aktivität von mindestens 2 E/mg Gesamtprotein, welche Lösung weniger als 10% an bekannten Stabilisatoren, ausgesucht aus der Gruppe der Zucker, Polyole, Aminosäuren, Peptide und Carbonsäuren, sowie weniger als 0,5 Mol Ammoniumsulfat pro Liter enthält, wobei das Erhitzen während einer Zeitdauer vorgenommen wird, die ausreicht, infektiöse Agentien zu inaktivieren, vorzugsweise während 30 min bis 100 h.

2. Virussichere Blutgerinnungsfaktor XIII-Präparation nach Anspruch 1, dadurch gekennzeichnet, daß in Anwesenheit eines Tensids, vorzugsweise eines nicht-ionischen Tensids, erhitzt worden ist.

3. Virussichere Blutgerinnungsfaktor XIII-Präparation nach Anspruch 1, dadurch gekennzeichnet, daß die Lösung im wesentlichen frei ist von bekannten Stabilisatoren.

4. Verfahren zur Herstellung einer virussicheren Blutgerinnungsfaktor XIII-Präparation, wobei infektiöse Agentien durch Erhitzen inaktiviert werden, dadurch gekennzeichnet, daß eine wässerige Lösung einer Faktor XIII-hältigen Lösung, die weniger als 10% an bekannten Stabilisatoren, ausgesucht aus der Gruppe der Zucker, Polyole, Aminosäuren, Peptide und Carbonsäuren, sowie weniger als 0,5 Mol Ammoniumsulfat pro Liter enthält und eine spezifische Aktivität von mindestens 2 Einheiten/mg Gesamtprotein aufweist, bei einer Temperatur von 40 bis 65°C während einer Zeitdauer, die ausreicht, infektiöse Agentien zu inaktivieren, vorzugsweise während 30 min bis 100 h, erhitzt wird.

5. Verfahren nach Anspruch 4, dadurch gekennzeichnet, daß in Anwesenheit eines Tensids, vorzugsweise eines nicht-ionischen Tensids, erhitzt wird.

6. Verfahren nach Anspruch 4, dadurch gekennzeichnet, daß die Lösung im wesentlichen frei ist von bekannten Stabilisatoren.

7. Verfahren nach einem der Ansprüche 4 bis 6, dadurch gekennzeichnet, daß bei einer Temperatur und einer Dauer erhitzt wird, daß die biologische Aktivität des Faktors XIII zu mehr als 50% erhalten bleibt.

8. Verfahren nach einem der Ansprüche 4 bis 7, gekennzeichnet durch die folgenden Maßnahmen:

   - Bereitstellen von Faktor XIII-hältiger COHN-I-Fraktion,
   - Fällung von Faktor XIII aus der COHN-I-Fraktion und Abtrennung des Niederschlages,
   - kurzzeitiges Erhitzen des gelösten Niederschlages und Abtrennung des denaturierten Fibrinogens,
   - Erhitzen der Faktor XIII-hältigen Lösung ohne Zusatz von Stabilisatoren bei einer Temperatur von 40 bis 60°C während einer Dauer von 30 min bis 2 h.

9. Verwendung der Blutgerinnungsfaktor XIII-Präparation nach Anspruch 1 zur Herstellung von Präparaten für die therapeutische und prophylaktische Anwendung, für die Herstellung von Gewebeklebstoffen und für diagnostische Zwecke.

10. Verwendung der virussicheren Blutgerinnungsfaktor XIII-Präparation nach Anspruch 1 zur Herstellung eines virussichern Gewebeklebstoff-Präparates als Zusatz zu einem bereits virusinaktivierten Fibrinogen-Präparat.

## Claims

1. A virus-safe blood coagulation factor XIII preparation, which is obtainable by heating an agueous solution comprising the blood coagulation factor XIII with a specific acitivity of at least 2 U/mg total protein, which solution contains less than 10 % of known stabilizers selected from the group of sugars, polyols, amino acids, peptides and carboxylic acids, as well as less than 0.5 mol of ammonium sulfate per liter, wherein heating is effected for a period of time sufficient to inactivate infectious agents, preferably for 30 min to 100 h.

2. A virus-safe blood coagulation factor XIII preparation according to claim 1, characterised in that heating has been effected in the presence of a tenside, preferably a non-ionic tenside.

3. A virus-safe blood coagulation factor XIII preparation according to claim 1, characterised in that the solution is substantially free from known stabilizers.

4. A method of producing a virus-safe blood coagulation factor XIII preparation, wherein infectious agents are inactivated by heating, characterised in that an agueous solution of a factor XIII-containing solution which contains less than 10% of known stabilisers selected from the group of sugars, polyols, amino acids, peptides and carboxylic acids, as well as less than 0.5 mol of ammonium sulfate per liter, and has a specific activity of at least 2 units/mg of total protein, is heated at a temperature of from 40 to 65°C for a period of time sufficient to inactivate infectious agents, preferably for 30 min to 100 h.

5. A method according to claim 4, characterised in that heating is effected in the presence of a tenside, preferably a non-ionic tenside.

6. A method according to claim 4, characterised in that the solution is substantially free from known stabilisers.

7. A method according to any one of claims 4 to 6, characterised in that heating is effected at a temperature and for a period of time at which more than 50% of the biologic activity of factor XIII is preserved.

8. A method according to any one of claims 4 to 7, characterised by the following measures:

   - providing a factor XIII-containing COHN I-fraction,
   - precipitating factor XIII from the COHN I-fraction and separating the precipitate,
   - short-term heating the dissolved precipitate and separating heat-denatured fibrinogen,
   - heating the factor XIII-containing solution without the addition of stabilizers at a temperature of from 40 to 60°C for a period of time of from 30 min to 2 h.

9. The use of the blood coagulation factor XIII preparation according to claim 1 for producing preparations for therapeutic and prophylactic applications, for the production of tissue adhesives and for diagnostic purposes.

10. The use of the virus-safe blood coagulation factor XIII preparation according to claim 1 for preparing a virus-safe tissue adhesive preparation as an addition to an already virus-inactivated fibrinogen preparation.

## Revendications

1. Préparation de facteur XIII de coagulation sanguine exempte de virus, pouvant être obtenue par chauffage d'une solution aqueuse contenant le facteur XIII de coagulation sanguine ayant une activité spécifique d'au moins 2 U/mg de protéine totale, cette solution contenant moins de 10 % de stabilisants connus choisis dans le groupe des sucres, des polyols, des aminoacides, des peptides et des acides carboxyliques, et moins de 0,5 mol de sulfate d'ammonium par litre, le chauffage étant effectué pendant une durée suffisante pour inactiver les agents infectieux, de préférence pendant 30 minutes à 100 heures.

2. Préparation de facteur XIII de coagulation sanguine exempte de virus selon la revendication 1, caractérisée en ce que le chauffage a été effectué en présence d'un agent tensioactif, de préférence d'un agent tensioactif non ionique.

3. Préparation de facteur XIII de coagulation sanguine exempte de virus selon la revendication 1, caractérisée en ce que la solution est essentiellement exempte de stabilisants connus.

4. Procédé de préparation d'une préparation de facteur XIII de coagulation sanguine exempte de virus, dans lequel les agents infectieux sont inactivés par chauffage, caractérisé en ce que l'on chauffe une solution aqueuse d'une solution contenant du facteur XIII, qui contient moins de 10 % de stabilisants connus choisis dans le groupe des sucres, des polyols, des aminoacides, des peptides et des acides carboxyliques, et moins de 0,5 mol de sulfate d'ammonium par litre, et qui a une activité spécifique d'au moins 2 unités/mg de protéine totale, à une température de 40 à 65°C pendant une durée suffisante pour l'inactivation des agents infectieux, de préférence pendant 30 minutes à 100 heures.

5. Procédé selon la revendication 4, caractérisé en ce que le chauffage s'effectue en présence d'un agent tensioactif, de préférence d'un agent tensioactif non ionique.

6. Procédé selon la revendication 4, caractérisé en ce que la solution est essentiellement exempte de stabilisants connus.

7. Procédé selon l'une des revendications 4 à 6, caractérisé en ce que l'on chauffe à une température et pendant un temps tels que l'activité biologique du facteur XIII est conservée à plus de 50 %.

8. Procédé selon l'une des revendications 4 à 7, caractérisé par les opérations suivantes:

   - Préparation d'une fraction de COHN I contenant du facteur XIII,
   - Précipitation du facteur XIII dans la fraction de COHN I et séparation du précipité,
   - Chauffage de courte durée du précipité dissous et séparation du fibrinogène dénaturé,
   - Chauffage de la solution contenant le facteur XIII sans addition de stabilisants connus à une température de 40 à 60°C pendant une durée de 30 minutes à 2 heures.

9. Utilisation de la préparation de facteur XIII de coagulation sanguine selon la revendication 1 pour la préparation de compositions à usage thérapeutique et prophylactique, pour la préparation de colles pour tissus et à des fins diagnostiques.

10. Utilisation de la préparation de facteur XIII de coagulation sanguine exempte de virus selon la revendication 1 pour la préparation d'une composition de colle pour tissus exempte de virus en tant qu'additif à une préparation de fibrinogène ayant déjà subi une inactivation des virus.